# EUROPEAN PATENT APPLICATION

(11) **EP 4 219 693 A1**
(43) Date of publication of application: **02.08.2023**
(21) Application number: 21888499.7
(22) Date of filing: 29.10.2021
(51) Int. Cl.: C12N 5/10, C12N 15/867, A61K 35/17, A61P 37/02

(54) **CHIMERIC ANTIGEN RECEPTOR TARGETING CD7 AND USE THEREOF**

(30) Priority: 03.11.2020 CN 202011209352
(71) Applicant: Bioheng Therapeutics Limited, Grand Cayman KY1-1209 (KY)
(72) Inventor: ZHOU, Yali, Nanjing, Jiangsu 210061 (CN); JIANG, Xiaoyan, Nanjing, Jiangsu 210061 (CN); CHEN, Gong, Nanjing, Jiangsu 210061 (CN); REN, Jiangtao, Nanjing, Jiangsu 210061 (CN); HE, Xiaohong, Nanjing, Jiangsu 210000 (CN); WANG, Yanbin, Nanjing, Jiangsu 210000 (CN); HAN, Lu, Nanjing, Jiangsu 210061 (CN)
(74) Representative: Rimini, Rebecca
(86) International application number: PCT/CN2021/127567
(87) International publication number: WO 2022/095802

(57) **Abstract**

Provided is an engineered immune cell. The cell expresses a chimeric antigen receptor comprising an antigen-binding region. The antigen-binding region comprises an anti-CD7 antibody, and the expression of endogenous CD7, at least one TCR/CD3 gene, and at least one MHC-II related gene is suppressed or silenced. Further provided is the use of the engineered immune cell in the treatment of diseases associated with CD7 expression.

## Description

### Technical Field

The present disclosure belongs to the field of immunotherapy. More specifically, the present disclosure relates to a CD7-targeting chimeric antigen receptor and use thereof in the treatment of diseases.

### Background Art

In recent years, cancer immunotherapy technology has developed rapidly. In particular, chimeric antigen receptor T cell (CAR-T)-related immunotherapy, as a new type of adoptive immunotherapy technology, has shown very significant clinical efficacy in the treatment of various solid tumors and hematological tumors.

According to the source of T cells used to construct CAR-T cells, CAR-T cells can be divided into autologous CAR-T cells and allogeneic CAR-T cells (also known as universal CAR-T). Autologous CAR-T cells have the advantage of avoiding the risk of immune rejection, because the initial T cells come from the patient receiving CAR-T therapy; but they also have very obvious disadvantages: high cost and long production cycle. Therefore, more and more researches focus on the development of universal CAR-T cells. Universal CAR-T can be prepared from T cells isolated from the peripheral blood of healthy donors, which greatly shortens the time for patients to wait for treatment. In addition, the viability and function of T cells obtained from healthy donors are also superior to that of T cells derived from patients, and can increase the infection rate of CAR cells and improve the therapeutic effect.

CD7 is a cell surface glycoprotein with a molecular weight of about 40 kDa and belongs to the immunoglobulin superfamily. CD7 is expressed in most T cells, NK cells, myeloid cells, T cell acute lymphoblastic leukemia/lymphoma, acute myelogenous leukemia and chronic myelogenous leukemia. It has been reported that the CD7 molecule acts as a co-stimulatory signal during T cell activation through the binding with its ligand K12/SECTM1. In addition, disruption of the CD7 molecule in mouse T progenitor cells still resulted in normal T cell development and homeostasis, suggesting that CD7 does not appear to have critical effects on T cell development and function, making it a very suitable therapeutic target for the treatment of T-cell acute lymphoblastic leukemia (T-ALL). Indeed, CD7 has been extensively studied as a target of cytotoxic molecules for the treatment of leukemia and lymphoma.

The current CAR-T therapy targeting CD7, especially the universal CAR-T therapy, is still very limited. Therefore, the present disclosure aims to provide a highly efficient universal CAR-T cell targeting CD7 and a use thereof in treating diseases.

### Summary

In a first aspect, the present disclosure provides an engineered immune cell, (1) expressing a chimeric antigen receptor comprising an antigen-binding region, the antigen-binding region comprising an anti-CD7 antibody; and (2) having suppressed or silenced expression of endogenous CD7, at least one TCR/CD3 gene, and at least one MHC-II related gene.

In an embodiment, the chimeric antigen receptor comprises the anti-CD7 antibody, a transmembrane domain, and an intracellular signaling domain.

In an embodiment, the anti-CD7 antibody comprises CDR-L1, CDR-L2 and CDR-L3 as set forth in SEQ ID NOs: 1, 2 and 3 respectively, and CDR-H1, CDR-H2 and CDR-H3 as set forth in SEQ ID NOs: 4, 5 and 6 respectively. In a preferred embodiment, the anti-CD7 antibody comprises a light chain variable region having at least 90% identity to an amino acid sequence selected from the group consisting of SEQ ID NOs: 7, 10, 13, 16 and 19, and a heavy chain variable region having at least 90% identity to an amino acid sequence selected from the group consisting of SEQ ID NOs: 8, 11, 14, 17 and 20. In a preferred embodiment, the amino acid sequence of the anti-CD7 antibody is selected from the group consisting of SEQ ID NOs: 9, 12, 15, 18 and 21.

In an embodiment, the antigen-binding region of the chimeric antigen receptor further comprises an antibody targeting a second antigen selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof. More preferably, the antibody targeting a second antigen is an antibody targeting CD19. In a preferred embodiment, the anti-CD19 antibody comprises a light chain variable region having at least 90% identity to an amino acid sequence selected from the group consisting of SEQ ID NOs: 52 and 55, and a heavy chain variable region having at least 90% identity to an amino acid sequence selected from the group consisting of SEQ ID NOs: 53 and 56. In a preferred embodiment, the amino acid sequence of the anti-CD19 antibody is selected from the group consisting of SEQ ID NOs: 54 and 57.

In an embodiment, the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154. Preferably, the transmembrane domain is a transmembrane domain of a protein selected from the group consisting of CD8 α, CD4 and CD28.

In an embodiment, the intracellular signaling domain is an intracellular region of a protein selected from the group consisting of: FcR γ, FcR β, CD3 γ, CD3 δ, CD3 ε, CD3 ζ, CD22, CD79a, CD79b, and CD66d. Preferably, the intracellular signaling domain comprises a CD3 ζ intracellular region.

In an embodiment, the chimeric antigen receptor further comprises at least one co-stimulatory domain, which is an intracellular region of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70 and a combination thereof. Preferably, the co-stimulatory domain is an intracellular region of CD27, CD28, CD134, CD137 or CD278 or a combination thereof.

In an embodiment, the TCR/CD3 gene is selected from the group consisting of TRAC, TRBC, CD3 γ, CD3 δ, CD3 ε, CD3 ζ and a combination thereof.

In an embodiment, the MHC-II related gene is selected from the group consisting of HLA-DPA, HLA-DQ, HLA-DRA, RFX5, RFXAP, RFXANK, CIITA and a combination thereof, preferably selected from the group consisting of RFX5, RFXAP, RFXANK, CIITA and a combination thereof.

In a preferred embodiment, the engineered immune cell of the present disclosure has suppressed or silenced expression of endogenous CD7, suppressed or silenced expression of at least one TCR/CD3 gene selected from the group consisting of TRAC and TRBC and suppressed or silenced expression of at least one MHC class II gene selected from the group consisting of RFX5, RFXAP, RFXANK and CIITA. More preferably, the engineered immune cell of the present disclosure has suppressed or silenced expression of endogenous CD7, at least one TCR/CD3 gene selected from the group consisting of TRAC and TRBC, and RFX5.

In an embodiment, the engineered immune cell further expresses a NK inhibitory molecule, which comprises one or more NK inhibitory ligands, a transmembrane domain and at least one co-stimulatory domain.

In an embodiment, the NK inhibitory ligand is an antibody targeting a NK inhibitory receptor selected from the group consisting of a NKG2/CD94 component (e.g., NKG2A, NKG2B, CD94); a member of the killer cell Ig-like receptor (KIR) family (e.g., KIR2DL1, KIR2DL2/3, KIR2DL5A, KIR2DL5B, KIR3DL1, KIR3DL2, and KIR3DL3); a member of the leukocyte Ig-like receptor (LIR) family (e.g., LIR1, LIR2, LIR3, LIR5, and LIR8); a member of the NK cell receptor protein 1 (NKR-P1) family (e.g., NKR-P1B and NKR-P1D); an immune checkpoint receptor (e.g., PD-1, TIGIT, and CD96, TIM3, LAG3); carcinoembryonic antigen-related cell adhesion molecule 1 (CEACAM1); a member of the sialic acid-binding immunoglobulin-like lectin (SIGLEC) family (e.g., SIGLEC7 and SIGLEC9); leukocyte-associated immunoglobulin-like receptor 1 (LAIR1); a member of the Ly49 family (e.g., Ly49A, Ly49C, Ly49F, Ly49G1 and Ly49G4) and killer cell lectin-like receptor G1 (KLRG1). Preferably, the NK inhibitory receptor is preferably selected from the group consisting of NKG2A, NKG2B, CD94, LIR1, LIR2, LIR3, LIR5, LIR8, KIR2DL1, KIR2DL2/3, KIR2DL5A, KIR2DL5B, KIR3DL1, KIR3DL2, KIR3DL3, CEACAM1, LAIR1, NKR-P1B, NKR-P1D, PD-1, TIGIT, CD96, TIM3, LAG3, SIGLEC7, SIGLEC9, Ly49A, Ly49C, Ly49F, Ly49G1, Ly49G4 and KLRG1. More preferably, the NK inhibitory receptor is selected from the group consisting of NKG2A, NKG2B, CD94, LIR1, LIR2, LIR3, KIR2DL1, KIR2DL2/3, KIR3DL1, CEACAM1, LAIR1 and KLRG1. Still more preferably, the NK inhibitory receptor is selected from the group consisting of NKG2A, NKG2B, LIR1, KIR2DL1, KIR2DL2/3, KIR3DL1, CEACAM1, LAIR1 and KLRG1.

In an embodiment, the NK inhibitory ligand is a natural ligand of a NK inhibitory receptor or a NK inhibitory receptor binding region comprised thereof. Preferably, the NK inhibitory ligand is selected from the group consisting of HLA-E, HLA-F, HLA-G, cadherin, collagen, OCIL, sialic acid, an immune checkpoint ligand (e.g., PD-L1/PD-L2, CTLA4, CD155, CD112, CD113, Gal-9, FGL1, etc.), and a NK inhibitory receptor binding region comprised thereof. More preferably, the NK inhibitory ligand is selected from the group consisting of HLA-E, HLA-F, HLA-G, cadherin, collagen, OCIL, sialic acid, PD-L1/PD-L2, CTLA-4, CD155, CD112, CD113, Gal-9, FGL1, and a NK inhibitory receptor binding region comprised thereof; more preferably selected from the group consisting of HLA-E, HLA-F, HLA-G, cadherin, PD-L1, PD-L2, and a NK inhibitory receptor binding region comprised thereof. In a preferred embodiment, the NK inhibitory ligand is selected from the group consisting of a HLA-E extracellular region, a HLA-G extracellular region, an E-cadherin extracellular region, a PD-L1 extracellular region and a PD-L2 extracellular region. In a preferred embodiment, the NK inhibitory ligand is an E-cadherin extracellular region comprising EC1 and EC2, more preferably comprising EC1, EC2, EC3, EC4 and EC5. In a preferred embodiment, the NK inhibitory ligand is an extracellular region of PD-L1 or PD-L2. In a preferred embodiment, the NK inhibitory ligand is a HLA-E extracellular region. In a preferred embodiment, the NK inhibitory ligand is a HLA-G extracellular region.

In an embodiment, the NK inhibitory molecule further comprises a CD3 ζ intracellular region as an intracellular signaling domain.

In an embodiment, the engineered immune cell is a T cell, a macrophage, a dendritic cell, a monocyte, a NK cell or a NKT cell. Preferably, the engineered immune cell is a T cell, such as CD4+/CD8+ T cell, CD4+ helper T cell (such as a Th1 or Th2 cell), CD8+ T cell (such as cytotoxic T cell), tumor infiltrating cell, memory T cell, naive T cell, γδ-T cell, αβ-T cell.

In an aspect, the present disclosure further provides a pharmaceutical composition, comprising the engineered immune cell described in the present disclosure as an active agent, and one or more pharmaceutically acceptable excipients.

In an aspect, the present disclosure further provide a method for treating a subject with a disease associated with CD7 expression, comprising administering to the subject an effective amount of the engineered immune cell or the pharmaceutical composition according to the present disclosure. Therefore, the present disclosure further encompasses the use of the engineered immune cell in the preparation of a medicine for treating a disease associated with CD7 expression.

### Detailed Description of Embodiments

Unless otherwise specified, all scientific and technical terms used herein have the same meaning as commonly understood by one of ordinary skill in the art to which this disclosure belongs.

### Chimeric antigen receptor

In a first aspect, the present disclosure provides an engineered immune cell, wherein the cell: (1) expresses a chimeric antigen receptor comprising an antigen-binding region, the antigen-binding region comprising an anti-CD7 antibody; and (2) has suppressed or silenced expression of endogenous CD7, at least one TCR/CD3 gene, and at least one MHC-II related gene.

As used herein, the term "chimeric antigen receptor" or "CAR" refers to an artificially constructed hybrid polypeptide, where the hybrid polypeptide generally includes an antigen-binding region (e.g., an antibody or antigen-binding portion thereof), a transmembrane domain, at least one optional co-stimulatory domain, and an intracellular signaling domain, and various domains are linked via a linker. CAR can redirect the specificity and reactivity of T cell and other immune cells to a selected target in a non-MHC-restricted manner by utilizing the antigen binding properties of antibodies. Non-MHC-restricted antigen recognition gives CAR-expressing immune cells the ability to recognize an antigen independent of antigen processing, thus bypassing the major mechanism of tumor escape.

In an embodiment, the CAR expressed by the engineered immune cells provided by the present disclosure comprises a CD7-targeting antibody or an antigen-binding fragment thereof, a transmembrane domain, and an intracellular signaling domain.

As used herein, the term "antibody" has the broadest meaning understood by those skilled in the art and includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity. The antibodies of the disclosure may be of any class (e.g., IgG, IgE, IgM, IgD, IgA, etc.) or subclass (e.g., IgG1, IgG2, IgG2a, IgG3, IgG4, lgA1, IgA2, etc.). Antibodies of the present disclosure also include recombinant antibodies, human antibodies, humanized antibodies, murine antibodies, chimeric antibodies, and antigen-binding portions thereof.

As used herein, "antibody fragment" or "antigen-binding portion" refers to a portion of an intact antibody, and typically comprises the antigen-binding site of the intact antibody and thus retains the ability to bind antigen. Examples of antibody fragments in the present disclosure include, but are not limited to: Fab, Fab', F(ab')2, Fd fragment, Fd', Fv fragment, scFv, disulfide-linked Fv (sdFv), heavy chain variable region (VH) or light chain variable region (VL) of an antibody, linear antibody, "diabody" with two antigen binding sites, single domain antibody, nanobody, a natural ligand for the antigen or a functional fragment thereof. Accordingly, an "antibody" of the present disclosure encompasses an antibody fragment or an antigen-binding portion of an antibody as defined above.

In an embodiment, the anti-CD7 antibody of the disclosure is an anti-CD7 scFv. "Single-chain antibody" and "scFv" are used interchangeably herein and refer to an antibody formed by linking the heavy chain variable region (VH) and the light chain variable region (VL) of an antibody through a linker. The optimal length and/or amino acid composition of the linker can be determined as desired. The length of the linker can significantly affect the folding and interaction of the variable domain of scFv. In fact, if shorter linkers (e.g., with between 5-10 amino acids) are used, intrachain folding can be prevented. For selection of linker size and composition, see, e.g., Hollinger et al., 1993 Proc Natl Acad. Sci. U.S.A. 90:6444-6448; U.S. Patent Application Publication Nos. 2005/0100543, 2005/0175606, 2007/0014794 and PCT Publication Nos. WO2006/020258 and WO2007/024715, the entire contents of which are incorporated herein by reference. A scFv may comprise VH and VL linked in any order, e.g. VH-linker-VL or VL-linker-VH.

In an embodiment, the CAR of the present disclosure comprises a CD7-targeting antibody, which comprises CDR-L1, CDR-L2 and CDR-L3 as set forth in SEQ ID NOs: 1, 2, and 3, respectively, and CDR-H1, CDR-H2 and CDR-H3 as set forth in SEQ ID NOs: 4, 5 and 6 respectively. Preferably, the CD7-targeting antibody of the present disclosure comprises a light chain variable region having at least 70%, preferably at least 80%, more preferably at least 90%, at least 95%, at least 97%, at least 99% or 100% sequence identity to an amino acid sequence selected from the group consisting of SEQ ID NOs: 7, 10, 13, 16 and 19, and a heavy chain variable region having at least 70%, preferably at least 80%, more preferably at least 90%, at least 95%, at least 97%, at least 99% or 100% sequence identity to an amino acid sequence selected from the group consisting of SEQ ID NOs: 8, 11, 14, 17 and 20. More preferably, the chimeric antigen receptor of the present disclosure comprises an anti-CD7 antibody having an amino acid sequence as set forth in SEQ ID NOs: 9, 12, 15, 18 or 21.

In an embodiment, in addition to the antibody targeting CD7, the antigen-binding region of the chimeric antigen receptor of the present disclosure comprises an antibody targeting a second antigen selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof. Preferably, the tumor antigen is selected from the group consisting of CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin, and any combination thereof. All antibodies known in the art targeting the above tumor antigens can be used in the present disclosure.

In a preferred embodiment, the antibody targeting a second antigen is an antibody targeting CD19, which comprises a light chain variable region having at least 70%, preferably at least 80%, more preferably at least 90%, at least 95%, at least 97%, at least 99% or 100% sequence identity to an amino acid sequence as set forth in SEQ ID NO: 52 or 55, and a heavy chain variable region having at least 70%, preferably at least 80%, more preferably at least 90%, at least 95%, at least 97%, at least 99% or 100% sequence identity to an amino acid sequence as set forth in SEQ ID NO: 53 or 56. More preferably, the chimeric antigen receptor of the present disclosure comprises a CD7-targeting antibody and a CD19-targeting antibody, and the amino acid sequence of the CD19-targeting antibody is set forth in SEQ ID NO: 54 or 57.

The term "functional variant" or "functional fragment" refers to a variant that substantially includes the amino acid sequence of a parent, but, compared with the parent amino acid sequence, comprises at least one amino acid modification (i.e., substitution, deletion, or insertion), provided that the variant retains the biological activity of the parent amino acid sequence. In an embodiment, the amino acid modification is preferably a conservative modification.

As used herein, the term "conservative modification" refers to amino acid modification that does not significantly affect or alter the binding characteristics of the antibody or antibody fragment comprising the amino acid sequence. These conservative modifications include amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is a substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with completely identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

As used herein, the term "transmembrane domain" refers to a polypeptide structure that enables expression of a chimeric antigen receptor on the surface of an immune cell (e.g., a lymphocyte, a NK cell, or a NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric antigen receptor binds to the target antigen. The transmembrane domains particularly suitable for use in the present disclosure may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CD8 α, CD9, CD16, CD22, CD33, CD28, CD37, CD64, CD80, CD86, CD134, CD137, CD154, and functional fragments thereof. Alternatively, the transmembrane domain may be synthesized and may mainly comprise hydrophobic residues such as leucine and valine. Preferably, the transmembrane domain is derived from a CD8 α chain or CD28, and has at least 70%, preferably at least 80%, more preferably at least 90%, least 95%, least 97% or least 99% or 100% sequence identity to an amino acid sequence as set forth in SEQ ID NO: 22 or 24, or the encoding sequence thereof has at least 70%, preferably at least 80%, more preferably at least 90%, least 95%, least 97% or least 99% or 100% sequence identity to a nucleotide sequence as set forth in SEQ ID NO: 23 or 25.

In an embodiment, the chimeric antigen receptor of the present disclosure may further comprise a hinge region located between the antigen-binding region and the transmembrane domain. As used herein, the term "hinge region" generally refers to any oligopeptide or polypeptide that functions to link a transmembrane domain to an antigen-binding region. Specifically, the hinge region serves to provide greater flexibility and accessibility to the antigen-binding region. The hinge region may comprise up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region comprises a hinge region portion of a CD8 α chain, CD28, an Fc γ RIII α receptor, an IgG4, or an IgG1, more preferably a hinge from CD8α, CD28 or IgG4, which has at least 70%, preferably at least 80%, more preferably at least 90%, least 95%, least 97% or least 99% or 100% sequence identity to an amino acid sequence as set forth in SEQ ID NO: 38, 40 or 42, or the encoding sequence thereof has at least 70%, preferably at least 80%, more preferably at least 90%, least 95%, least 97% or least 99% or 100% sequence identity to a nucleotide sequence as set forth in SEQ ID NO: 39, 41 or 43.

As used herein, the term "intracellular signaling domain" refers to a protein portion that transduces an effector function signal and guides a cell to perform a specified function. The intracellular signaling domain is responsible for intracellular primary signaling after the antigen-binding region binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, including secretion of cytokines.

In an embodiment, the intracellular signaling domain contained in the chimeric antigen receptor of the present disclosure may be cytoplasmic sequences of a T cell receptor and a co-receptor, upon antigen receptor binding, which act together to initiate primary signaling, as well as any derivative or variant of these sequences and any synthetic sequence having the same or similar function. The intracellular signaling domain may comprise many immunoreceptor tyrosine-based activation motifs (ITAM). Non-limiting examples of intracellular signaling domain of the present disclosure include, but are not limited to, the intracellular regions of FcR γ, FcR β, CD3 γ, CD3 δ, CD3 ε, CD3 ζ, CD22, CD79a, CD79b, and CD66d. In a preferred embodiment, the signaling domain of the CAR of the present disclosure may comprise a CD3 ζ intracellular region, which has at least 70%, preferably at least 80%, more preferably at least 90%, at least 95%, at least 97%, or at least 99% or 100% sequence identity to an amino acid sequence as set forth in SEQ ID NO: 30 or 32, or the encoding sequence thereof has at least 70%, preferably at least 80%, more preferably at least 90%, at least 95%, at least 97%, or at least 99% or 100% sequence identity to a nucleotide sequence as set forth in SEQ ID NO: 31 or 33.

In an embodiment, the chimeric antigen receptor of the present disclosure comprises one or more co-stimulatory domains. The co-stimulatory domain may be an intracellular functional signaling domain from a co-stimulatory molecule, which comprises an entire intracellular portion of the co-stimulatory molecule, or a functional fragment thereof. "Co-stimulatory molecule" refers to a homologous binding partner that specifically binds to a co-stimulatory ligand on a T cell, thereby mediating a co-stimulatory response (e.g. proliferation) of the T cell. The co-stimulatory molecule includes, but is not limited to, MHC class 1 molecules, BTLA, and Toll ligand receptors. Non-limiting examples of the co-stimulatory domain of the present disclosure include, but are not limited to, intracellular regions of the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM1), CD83, CD134 (OX40), CD137 (4-1BB), CD270 (HVEM), CD272 (BTLA) , CD276 (B7-H3), CD278 (ICOS), CD357 (GITR), DAP10, DAP12, LAT, NKG2C, SLP76, PD-1, LIGHT, TRIM, CD94, LTB and ZAP70, and combinations thereof.

In a preferred embodiment, the co-stimulatory domain comprises one or more intracellular regions of a protein selected from the group consisting of DAP10, DAP12, CD27, CD28, CD134, 4-1BB or CD278. For example, in an embodiment, the co-stimulatory domain comprises the intracellular region of 4-1BB. In an embodiment, the co-stimulatory domain comprises the intracellular region of CD28. In an embodiment, the co-stimulatory domain comprises the intracellular region of 4-1BB and the intracellular region of CD28.

In an embodiment, the intracellular region of 4-1BB has at least 70%, preferably at least 80%, more preferably at least 90%, at least 95%, at least 97%, or at least 99% or 100% sequence identity to an amino acid sequence as set forth in SEQ ID NO: 28, or the encoding sequence thereof has at least 70%, preferably at least 80%, more preferably at least 90%, at least 95%, at least 97%, or at least 99% or 100% sequence identity to a nucleotide sequence as set forth in SEQ ID NO: 29. In an embodiment, the intracellular region of CD28 has at least 70%, preferably at least 80%, more preferably at least 90%, at least 95%, at least 97%, or at least 99% or 100% sequence identity to an amino acid sequence as set forth in SEQ ID NO: 26, or the encoding sequence thereof has at least 70%, preferably at least 80%, more preferably at least 90%, at least 95%, at least 97%, or at least 99% or 100% sequence identity to a nucleotide sequence as set forth in SEQ ID NO: 27.

In an embodiment, the CAR of the present disclosure may further comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to those skilled in the art, for example, signal peptides derived from CDS α, IgG1, GM-CSFRα, B2M and so on. In an embodiment, the signal peptide that can be used in the present disclosure is from B2M or CD8α, which has at least 70%, preferably at least 80%, more preferably at least 90%, at least 95%, at least 97%, or at least 99% or 100% sequence identity to an amino acid sequence as set forth in SEQ ID NO: 34 or 36, or the encoding sequence thereof has at least 70%, preferably at least 80%, more preferably at least 90%, at least 95%, at least 97%, or at least 99% or 100% sequence identity to a nucleotide sequence as set forth in SEQ ID NO: 35 or 37.

In an embodiment, the CAR of the present disclosure may further comprise a switch structure to regulate the expression time of the CAR. For example, the switch structure may be in a form of dimerization domain, which causes a conformational change by binding to a corresponding ligand thereof, and exposes the extracellular antigen-binding region to enable its binding to a targeted antigen, thereby activating a signaling pathway. Alternatively, a switch structure also may be used to link the antigen-binding region and signaling domain, respectively, and only when the switch structures are bound to each other (for example, in the presence of an inducing compound), the antigen-binding region and the signaling domain can be linked together through a dimer, thereby activating signaling pathway. The switch structure also can be in the form of a masking peptide. The masking peptide can shield the extracellular antigen-binding region, and prevent it from binding to the targeted antigen. When the masking peptide is cleaved by, for example, a protease, the extracellular antigen-binding region is exposed, making it become a "normal" CAR structure. A variety of switch structures known to those skilled in the art can be used in the present disclosure.

In an embodiment, the CAR of the present disclosure may further comprise a suicide gene, to make it express a cell death signal that can be induced by an exogenous substance, so as to eliminate the CAR cell when needed (e.g., when serious toxic side effects are produced). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce the cell to die when receiving ganciclovir treatment. The suicide gene may further be iCaspase-9, and dimerization of iCaspase-9 can be induced by a chemical induction drug such as AP1903 and AP20187, so as to activate the downstream Caspase3 molecule, and cause apoptosis. A variety of suicide genes known to those of skill in the art can be used in the present disclosure.

### Inhibition or silencing of endogenous gene expression

In an embodiment, the engineered immune cell provided by the present disclosure has suppressed or silenced expression of endogenous CD7, at least one TCR/CD3 gene, and at least one MHC-II related gene.

CD7 is not only expressed in tumor cells, such as T cell acute lymphoblastic leukemia/lymphoma, acute myelogenous leukemia and chronic myelogenous leukemia, but also expressed in most normal T cells and NK cells. Therefore, in order to avoid mutual recognition and killing between CAR cells targeting CD7, it is necessary to inhibit or silence the expression of the endogenous CD7 gene of the CAR cells.

T cell receptor (TCR) is a characteristic mark on the surface of all T cells. It binds to CD3 through a non-covalent bond to form a TCR/CD3 complex, and binds to the specific MHC-antigen peptide complex on the surface of antigen-presenting cells to generate specific antigen stimulation signals, to activate T cells to play a killing role. Therefore, inhibiting or silencing the expression of the endogenous TCR/CD3 gene in a CAR-T cell can avoid its attack on normal cells or tissues in patients, thereby avoiding or reducing the risk of graft-versus-host disease (GvHD). TCR is a heterodimer composed of two different peptide chains, and usually divided into two types: α/β type and γ/δ type. More than 95% of peripheral T lymphocytes express TCR α/β. The TCR alpha chain is encoded by the TRAC gene, and the beta chain is encoded by the TRBC gene. Each peptide chain of TCR includes a variable region (V region), a constant region (C region), a transmembrane region and a cytoplasmic region, wherein the cytoplasmic region is very short and does not have the ability to transmit antigen stimulation signals. The TCR molecule belong to the immunoglobulin superfamily, and its antigen specificity exists in the V region; the V region has three hypervariable regions, CDR1, CDR2, and CDR3, among which CDR3 is the most variable, and directly determines the antigen-binding specificity of TCR. When TCR recognizes the MHC-antigen peptide complex, CDR1 and CDR2 recognize and bind to MHC molecules, while CDR3 directly binds to the antigen peptide. CD3 includes four subunits: γ, δ, ε, ζ, usually in the form of dimers εγ, εδ, ζζ. These four subunits all comprise the conserved immunoreceptor tyrosine-based activation motif (ITAM), and after the 2 tyrosine residues are phosphorylated by tyrosine protein kinase, they transmit activation signals to T cells. Thus, in an embodiment, the at least one TCR/CD3 gene is selected from the group consisting of: TRAC, TRBC, CD3 γ, CD3 δ, CD3 ε, CD3 ζ.

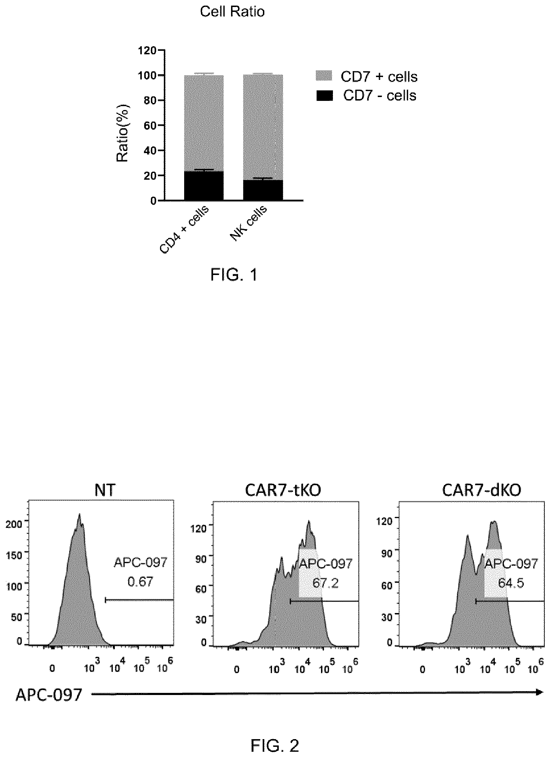

By detecting the composition of peripheral blood lymphocytes in patients, the inventors found that CD4+CD7- T cells accounted for about 20% of the total CD3+ T cells (Figure 1). Since CAR cells targeting CD7 cannot recognize the CD4+CD7- T population, they cannot effectively kill this population. Instead, CD4+CD7- T cells will kill exogenous CAR cells by recognizing MHC-II molecules. Therefore, in order to avoid the killing of exogenous CAR-T cells by CD4+CD7- T cells in patients, the inventors consider inhibiting or silencing the expression of endogenous MHC-II related genes in CAR-T cells.

In the present disclosure, MHC-II related genes include MHC-II genes themselves, and genes that interact with MHC-II genes or regulate their expression.

The major histocompatibility complex (MHC), originally characterized as a protein that plays a major role in the transplant response, is expressed on the surface of all higher vertebrates and is called H-2 in mice, called HLA in human cells. There are two main classes of MHC: class I and class II. The MHC class I protein is a heterodimer of two proteins: one is the alpha chain of a transmembrane protein encoded by the MHC I gene, and the other is the beta2 microglobulin chain of an extracellular protein encoded by a gene not located within the MHC gene cluster. The alpha chain comprises three domains and the foreign peptide binds to two domains α1, α2 which are also the most variable at the N-terminus. The MHC class II protein is also a heterodimer, comprising two transmembrane proteins encoded by genes within the MHC complex. Class I MHC/antigen complexes interact with cytotoxic T cells (e.g., CD8+ T cells), while class II MHC present antigens to helper T cells (e.g., CD4+ T cells). Furthermore, MHC class I proteins tend to be expressed in almost all nucleated cells and platelets (and red blood cells in mice), whereas MHC class II proteins are more selectively expressed. Typically, MHC class II proteins are expressed on B cells, some macrophages and monocytes, activated T cells, Langerhans cells and dendritic cells.

The human HLA class II cluster comprises three major loci DP, DQ and DR. The Class II molecule is a heterodimer consisting of an alpha chain and a beta chain, both anchored in the membrane, where the alpha chain is approximately 33-35 kDa and comprises 5 exons. Exon 1 encodes the leader peptide, exons 2 and 3 encode the two extracellular domains, exon 4 encodes the transmembrane domain, and exon 5 encodes the cytoplasmic tail. Thus, in an embodiment, the MHC class II related gene is selected from the group consisting of: HLA-DPA, HLA-DQ and HLA-DRA.

The expression of MHC-II genes also depends on a variety of important positive regulatory proteins, such as RFX complex, CIITA and so on. The RFX complex is composed of three subunits: RFXANK (also known as RFXB), RFX5 and RFX accessory protein (also known as RFXAP). The RFX complex promotes the expression of MHC class II molecules by facilitating the binding of other transcription factors to the promoters of MHC class II molecules and enhancing the specificity of promoter binding. CIITA is a master controller of MHC class II expression. CIITA includes an N-terminal rich in acidic amino acids, a PST region rich in Pro, Ser, and Thr, a GTP-binding region in the middle, and a C-terminal rich in Leu repeat sequence (LRR), where the N-terminal acidic region and PST region are transcriptional activation area. Thus, in an embodiment, the MHC class II related gene is selected from the group consisting of: RFX5, RFXAP, RFXANK and CIITA.

Therefore, in an embodiment, the MHC class II related gene is selected from the group consisting of HLA-DPA, HLA-DQ, HLA-DRA, RFX5, RFXAP, RFXANK and CIITA, preferably selected from the group consisting of RFX5, RFXAP, RFXANK and CIITA, more preferably RFX5.

In an embodiment, the endogenous MHC class I gene (e.g., HLA-A, HLA-B, HLA-C, B2M, etc.) in the CAR-T cell is functional. In another embodiment, the expression of endogenous MHC-class I gene in CAR-T cells is also suppressed or silenced.

In an embodiment, the engineered immune cell expressing a CD7-targeting chimeric antigen receptor according to the present disclosure has suppressed or silenced expression of endogenous CD7, at least one TCR/CD3 gene, and at least one MHC-II related gene, wherein the at least one TCR/CD3 gene is selected from the group consisting of TRAC, TRBC, CD3 γ, CD3 δ, CD3 ε, CD3 ζ and a combination thereof; and the at least one MHC-II related gene is selected from the group consisting of HLA-DPA, HLA-DQ, HLA-DRA, RFX5, RFXAP, RFXANK, and CIITA. In a preferred embodiment, the engineered immune cell expressing a CD7-targeting chimeric antigen receptor according to the present disclosure has suppressed or silenced expression of endogenous CD7, at least one TCR/CD3 gene selected from the group consisting of TRAC and TRBC and at least one MHC class II gene selected from the group consisting of RFX5, RFXAP, RFXANK and CIITA. More preferably, the engineered immune cell expressing a CD7-targeting chimeric antigen receptor according to the present disclosure has suppressed or silenced expression of endogenous CD7, at least one TCR/CD3 gene selected from the group consisting of TRAC and TRBC, and RFX5. In an embodiment, the expression of MHC class I genes such as HLA-A, HLA-B, HLA-C and B2M in the engineered immune cells is functional.

In an embodiment, in addition to CD7, MHC-II-related genes and TCR/CD3 genes, the engineered immune cell of the present disclosure may further has suppressed or silenced expression of at least one gene selected from the group consisting of: CD52, GR, dCK and an and immune checkpoint gene such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for inhibiting gene expression or for gene silencing are well known to those skilled in the art, including but not limited to, for example, DNA fragmentation mediated by meganucleases, zinc finger nucleases, TALE nucleases, or Cas enzymes in the CRISPR system, or gene inactivation by antisense oligonucleotides, RNAi, shRNA, and other technologies.

### NK inhibitory molecule

The inventors also found that there is a large proportion (about 20%) of CD7- NK cells in patients (Figure 1), which cannot be targeted and killed by CD7 CAR. Moreover, when the expression of MHC-II gene is suppressed or silenced, these cells may kill CAR cells.

Therefore, in an embodiment, in order to inhibit the killing of CAR-T cells by NK cells in the patient, the engineered immune cell further express a NK inhibitory molecule, which comprises one or more NK inhibitory ligands, a transmembrane domain and a co-stimulatory domain. For example, the NK inhibitory molecule comprises one or two NK inhibitory ligands, a transmembrane domain and a co-stimulatory domain.

The transmembrane domain and co-stimulatory domain contained in the NK inhibitory molecule have the same definitions as those contained in the chimeric antigen receptor as described above.

In an embodiment, the NK inhibitory ligand is an antibody targeting a NK inhibitory receptor selected from the group consisting of a NKG2/CD94 component (e.g., NKG2A, NKG2B, CD94); a member of the killer cell Ig-like receptor (KIR) family (e.g., KIR2DL1, KIR2DL2/3, KIR2DL5A, KIR2DL5B, KIR3DL1, KIR3DL2, and KIR3DL3); a member of the leukocyte Ig-like receptor (LIR) family (e.g., LIR1, LIR2, LIR3, LIR5, and LIR8); a member of the NK cell receptor protein 1 (NKR-P1) family (e.g., NKR-P1B and NKR-P1D); an immune checkpoint receptor (e.g., PD-1, TIGIT, and CD96, TIM3, LAG3); carcinoembryonic antigen-related cell adhesion molecule 1 (CEACAM1); a member of the sialic acid-binding immunoglobulin-like lectin (SIGLEC) family (e.g., SIGLEC7 and SIGLEC9); leukocyte-associated immunoglobulin-like receptor 1 (LAIR1); a member of the Ly49 family (e.g., Ly49A, Ly49C, Ly49F, Ly49G1 and Ly49G4) and killer cell lectin-like receptor G1 (KLRG1). Preferably, the NK inhibitory receptor is preferably selected from the group consisting of NKG2A, NKG2B, CD94, LIR1, LIR2, LIR3, LIR5, LIR8, KIR2DL1, KIR2DL2/3, KIR2DL5A, KIR2DL5B, KIR3DL1, KIR3DL2, KIR3DL3, CEACAM1, LAIR1, NKR-P1B, NKR-P1D, PD-1, TIGIT, CD96, TIM3, LAG3, SIGLEC7, SIGLEC9, Ly49A, Ly49C, Ly49F, Ly49G1, Ly49G4 and KLRG1. More preferably, the NK inhibitory receptor is selected from the group consisting of NKG2A, NKG2B, CD94, LIR1, LIR2, LIR3, KIR2DL1, KIR2DL2/3, KIR3DL1, CEACAM1, LAIR1 and KLRG1. Still more preferably, the NK inhibitory receptor is selected from the group consisting of NKG2A, NKG2B, LIR1, KIR2DL1, KIR2DL2/3, KIR3DL1, CEACAM1, LAIR1 and KLRG1.

In an embodiment, the NK inhibitory ligand is an antibody targeting a NK inhibitory receptor, said antibody being a whole antibody, Fab, Fab', F(ab')2, a Fv fragment, a scFv antibody fragment, a linear antibody, a sdAb or a nanobody.

In a preferred embodiment, the NK inhibitory ligand is an antibody targeting NKG2A. More preferably, the antibody targeting NKG2A comprises a light chain variable region having at least 70%, preferably at least 80%, more preferably at least 90%, at least 95%, at least 97%, at least 99% or 100% sequence identity to an amino acid sequence as set forth in SEQ ID NO: 59, 62 or 77, and a heavy chain variable region having at least 70%, preferably at least 80%, more preferably at least 90%, at least 95%, at least 97%, at least 99% or 100% sequence identity to an amino acid sequence as set forth in SEQ ID NO: 58, 61 or 76. More preferably, the amino acid sequence of the antibody targeting NKG2A is set forth in SEQ ID NO: 60, 63 or 78. Other antibodies targeting NKG2A known in the art can also be used in the present disclosure, such as Z270 (available from Immunotech, France), Z199 (available from Beckman Coulter, USA), 20D5 (available from BD Biosciences Pharmingen, USA), P25 (available from Morettaetal, Univ. Genova, Italy) and the like.

In a preferred embodiment, the NK inhibitory ligand is an antibody targeting KIR, e.g. KIR2DL1, KIR2DL2/3 and KIR3DL1. More preferably, the antibody targeting KIR comprises a light chain variable region having at least 70%, preferably at least 80%, more preferably at least 90%, at least 95%, at least 97%, at least 99% or 100% sequence identity to an amino acid sequence as set forth in SEQ ID NO: 65 or 79, and a heavy chain variable region having at least 70%, preferably at least 80%, more preferably at least 90%, at least 95%, at least 97%, at least 99% or 100% sequence identity to an amino acid sequence as set forth in SEQ ID NO: 66 or 80. More preferably, the amino acid sequence of the antibody targeting KIR is set forth in SEQ ID NO: 67 or 81. Other antibodies targeting KIR known in the art can also be used in the present disclosure, such as GL183 (targeting KIR2DL2/L3, available from Immunotech, France and Beckton Dickinson, USA), EB6 (targeting KIR2DL1, available from Immunotech, France and Beckton Dickinson, USA), AZ138 (targeting KIR3DL1, available from Morettaetal, Univ. Genova, Italy), Q66 (targeting KIR3DL2, available from Immunotech, France), Z27 (targeting KIR3DL1, available from Immunotech, France and Beckton Dickinson, USA) and the like.

In a preferred embodiment, the NK inhibitory ligand is an antibody targeting LIR1. More preferably, the antibody targeting LIR1 comprises a light chain variable region having at least 70%, preferably at least 80%, more preferably at least 90%, at least 95%, at least 97%, at least 99% or 100% sequence identity to an amino acid sequence as set forth in SEQ ID NO: 68 or 71, and a heavy chain variable region having at least 70%, preferably at least 80%, more preferably at least 90%, at least 95%, at least 97%, at least 99% or 100% sequence identity to an amino acid sequence as set forth in SEQ ID NO: 69 or 72. More preferably, the amino acid sequence of the antibody targeting LIR1 is set forth in SEQ ID NO: 70 or 73.

In an embodiment, the NK inhibitory ligand is a natural ligand of a NK inhibitory receptor or a NK inhibitory receptor binding region comprised thereof. Preferably, the NK inhibitory ligand is selected from the group consisting of HLA-E, HLA-F, HLA-G, cadherin, collagen, OCIL, sialic acid, an immune checkpoint ligand (e.g., PD-L1/PD-L2, CD155, CD112, CD113, Gal-9, FGL1, etc.), and a NK inhibitory receptor binding region comprised thereof. More preferably, the NK inhibitory ligand is selected from the group consisting of HLA-E, HLA-F, HLA-G, cadherin, collagen, OCIL, sialic acid, PD-L1/PD-L2, CTLA-4, CD155, CD112, CD113, Gal-9, FGL1, and a NK inhibitory receptor binding region comprised thereof; more preferably selected from the group consisting of HLA-E, HLA-F, HLA-G, cadherin, and a NK inhibitory receptor binding region comprised thereof. More preferably, the NK inhibitory ligand is selected from the group consisting of a HLA-E extracellular region, a HLA-G extracellular region, an E-cadherin (E-Cadherin) extracellular region, a PD-L1 extracellular region and a PD- L2 extracellular region.

In a preferred embodiment, the NK inhibitory ligand is an E-cadherin extracellular region comprising domains EC1 and EC2, more preferably comprising domains EC1, EC2, EC3, EC4 and EC5. Preferably, the E-cadherin extracellular region has at least 70%, preferably at least 80%, more preferably at least 90%, at least 95%, at least 97%, at least 99% or 100% sequence identity to an amino acid sequence as set forth in SEQ ID NO: 44 (comprising domains EC1 and EC2) or SEQ ID NO: 48 (comprising domains EC1, EC2, EC3, EC4 and EC5).

In an embodiment, the NK inhibitory ligand is a PD-L1 or PD-L2 extracellular region. Preferably, the PD-L1 extracellular region has at least 70%, preferably at least 80%, more preferably at least 90%, at least 95%, at least 97%, at least 99% or 100% sequence identity to an amino acid sequence as set forth in SEQ ID NO: 45, and the PD-L2 extracellular region has at least 70%, preferably at least 80%, more preferably at least 90%, at least 95%, at least 97%, at least 99% or 100% sequence identity to an amino acid sequence as set forth in SEQ ID NO: 46.

In an embodiment, the NK inhibitory ligand is a HLA-E extracellular region or a HLA-G extracellular region. Preferably, the HLA-E extracellular region has at least 70%, preferably at least 80%, more preferably at least 90%, at least 95%, at least 97%, at least 99% or 100% sequence identity to an amino acid sequence as set forth in SEQ ID NO: 50 (wild type) or SEQ ID NO: 51 (a mutant comprising Y84C mutation), and the HLA-G extracellular region has at least 70%, preferably at least 80%, more preferably at least 90%, at least 95%, at least 97%, at least 99% or 100% sequence identity to an amino acid sequence as set forth in SEQ ID NO: 49. It is known in the art that non-classical HLA-class I molecules (such as HLA-E, HLA-G) need to form a complex with B2M to exert their functions. Therefore, when B2M needs to be knocked out, in order for non-classical HLA class I molecules to exert their inhibitory function normally, it is necessary to introduce B2M with synonymous mutations in the DNA sequence to avoid being knocked out by gene editing tools. In other words, in B2M-knockout cells, the NK inhibitory ligand is a fusion molecule of B2M and the extracellular region of HLA-E or the extracellular region of HLA-G.

In yet another embodiment, the NK inhibitory molecule comprises at least two NK inhibitory ligands selected from the group consisting of anti-NKG2A scFv, anti-KIR scFv, anti-LIR1 scFv, HLA-E extracellular region, HLA-G extracellular region, E-cadherin extracellular region, PD-L1 extracellular region and PD-L2 extracellular region, more preferably PD-L1 extracellular region and HLA-E extracellular region.

In an embodiment, the NK inhibitory molecule further comprises an intracellular signaling domain. In an embodiment, the intracellular signaling domain is an intracellular region of a protein selected from the group consisting of: FcR γ, FcR β, CD3 γ, CD3 δ, CD3 ε, CD3 ζ, CD22, CD79a, CD79b, and CD66d. Preferably, the intracellular signaling domain comprises a CD3 ζ intracellular region.

In an embodiment, the NK inhibitory molecule may further comprise a signal peptide, such as a signal peptide from PDL1 or B2M. In an embodiment, the NK inhibitory molecule comprises a PD-L1 signal peptide, which has at least 70%, preferably at least 80%, more preferably at least 90%, at least 95%, at least 97%, at least 99% or 100% sequence identity to an amino acid sequence as set forth in SEQ ID NO: 44. Those skilled in the art can also select other suitable signal peptides as needed.

### Engineered immune cell

The present disclosure further provides an engineered immune cell, wherein the cell: (1) expresses a chimeric antigen receptor comprising an antigen-binding region, the antigen-binding region comprising an anti-CD7 antibody and an optional second antigen region; and (2) has suppressed or silenced expression of endogenous CD7, at least one TCR/CD3 gene, and at least one MHC-II related gene. In an embodiment, the engineered immune cell of the present disclosure further expresses a NK inhibitory molecule comprising one or more NK inhibitory ligands, a transmembrane domain and a co-stimulatory domain.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, a NK cell, and/or a NKT cell, an immune cell derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell may be any T cell, such as *in vitro* cultured T cell, for example, primary T cell, or T cell from *in vitro* cultured T cell line, e.g., Jurkat, SupT1, etc., or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be at any stage of development including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell. In a preferred embodiment, the immune cell is a human T cell. The T cell can be isolated from the blood of a subject using a variety of techniques known to those of skill in the art, such as Ficoll. In the present disclosure, the immune cell is engineered to express a chimeric antigen receptor and to suppress or silence the expression of endogenous CD7, at least one TCR/CD3 gene, and at least one MHC class II related gene.

The nucleic acid sequence encoding the chimeric antigen receptor polypeptide and an optional nucleic acid sequence encoding the NK inhibitory molecule can be introduced into the immune cell by conventional methods known in the art (e.g., by transduction, transfection, transformation). "Transfection" is a process of introducing a nucleic acid molecule or polynucleotide (including a vector) into a target cell. An example is RNA transfection, i.e., the process of introducing RNA (e.g., *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake through a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation.

After the nucleic acid or vector is introduced into the immune cells, those skilled in the art can amplify and activate the obtained immune cells by conventional techniques.

### Pharmaceutical composition

The present disclosure further provides a pharmaceutical composition, comprising the engineered immune cell described in the present disclosure as an active agent, and one or more pharmaceutically acceptable excipients.

As used herein, the term "pharmaceutically acceptable excipient" refers to a carrier and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the application is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present disclosure may further be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulatory agents such as platelet factor 4, and melanoma growth stimulating protein and so on; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic use

The present disclosure further provides a method of treating a subject with a disease associated with CD7 expression, comprising administering to the subject an effective amount of the immune cell or the pharmaceutical composition according to the present disclosure. Therefore, the present disclosure also encompasses use of the engineered immune cell and the pharmaceutical composition in the preparation of a medicine for treating diseases associated with CD7 expression.

In an embodiment, an effective amount of the immune cell and/or the pharmaceutical composition of the present disclosure is directly administered to the subject.

In another embodiment, the treatment method of the present disclosure is *ex vivo* treatment. Specifically, the method includes the steps of: (a) providing a sample, the sample containing an immune cell; (b) introducing the chimeric antigen receptor of the present disclosure and an exogenous gene (such as for a NK inhibitory molecule) into the immune cell *in vitro,* and suppressing or silencing the expression of specific genes (such as endogenous CD7, TCR/CD3 genes and MHC-II related genes) in the immune cell to obtain a modified immune cell, and (c) administering the modified immune cell to a subject in need thereof. Preferably, the immune cell provided in step (a) is selected from the group consisting of a macrophage, a dendritic cell, a monocyte, a T cell, a NK cell, and/or a NKT cell; and the immune cell can be obtained from a sample (particularly a blood sample) of the subject by conventional methods known in the art. However, other immune cells capable of expressing the chimeric antigen receptor of the present disclosure and exerting the desired biological effect function as described herein also can be used. Besides, the immune cells generally selected are compatible with the subject's immune system, i.e., it is preferred that the immune cells do not trigger an immunogenic response. For example, a "universal recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an autologous or allogeneic cell, preferably T cell, macrophage, dendritic cell, monocyte, NK cell and/or NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic material from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse, or a cow. Mammals other than human can be advantageously used as subjects representing cancer animal models. Preferably, the subject is a human.

In an embodiment, diseases associated with CD7 expression include non-solid tumors (such as hematological tumors, e.g., leukemias and lymphomas) and solid tumors. Hematological neoplasms are cancers of the blood or bone marrow, including but not limited to acute leukemias (such as acute lymphoblastic leukemia (ALL), acute myeloid leukemia (AML), acute myelogenous leukemia, and myeloblastic, promyelocytic, myelomonocytic, monocytic and erythroleukemia), chronic leukemia (such as chronic myeloid (granulocytic) leukemia, chronic myelogenous leukemia, and chronic lymphocytic leukemia), polycythemia vera, lymphoma, Hodgkin's lymphoma, non-Hodgkin's lymphoma (indolent and high-grade forms), multiple myeloma, Waldenstrom's macroglobulinemia, myelodysplastic syndrome, hairy cell leukemia, Burkitt's lymphoma, diffuse large cell lymphoma, mantle cell lymphoma, T-lymphoblastic lymphoma (T-LBL), early pro-T-lymphoblastic leukemia (ETP-ALL), extranodal NK/T-cell lymphoma, small lymphocytic lymphoma (SLL) and myelodysplasia. Solid tumors are abnormal masses of tissue that usually do not comprise cysts or areas of fluid, and can be benign or malignant. The different types of solid tumors are named according to the type of cells that form them (such as sarcomas, carcinomas, and lymphomas). Examples of solid tumors include, but are not limited to, fibrosarcoma, myxosarcoma, liposarcoma, mesothelioma, pancreatic cancer, ovarian cancer, peritoneal, omental, and mesenteric cancer, pharyngeal cancer, prostate cancer, rectal cancer, renal cancer, skin cancer, small intestine cancer, melanoma, kidney cancer, laryngeal cancer, soft tissue cancer, stomach cancer, testicular cancer, colon cancer, esophagus cancer, cervical cancer, alveolar rhabdomyosarcoma, bladder cancer, bone cancer, brain cancer, breast cancer, anal cancer, eye cancer, intrahepatic cholangiocarcinoma, joint cancer, neck cancer, gallbladder cancer, pleural cancer, nasal cancer, middle ear cancer, oral cancer, vulvar cancer, thyroid cancer and ureter cancer.

In an embodiment, the disease associated with CD7 expression is preferably selected from the group consisting of acute lymphoblastic leukemia (ALL), acute myeloid leukemia (AML), T-lymphoblastic lymphoma (T-LBL), early pro-T lymphoblastic Leukemia (ETP-ALL) and extranodal NK/T cell lymphoma.

The present disclosure will be described in detail below with reference to the accompanying drawings and examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Brief Description of Drawings

Figure 1 shows the ratio of CD7+ cells and CD7- cells in peripheral blood lymphocytes of patients.
Figure 2 shows the expression levels of scFv on the CAR7-dKO T cells and CAR7-tKO T cells .
Figure 3 shows the killing ability of CAR7-dKO T cells and CAR7-tKO T cells on target cells.
Figure 4 shows the cytokine release levels after CAR7-dKO T cells and CAR7-tKO T cells were co-cultured with target cells.
Figure 5 shows the inhibitory effect of NK inhibitory molecules on NK cell killing ability. A: a NK inhibitory molecule comprises anti-KIR scFv; B: a NK inhibitory molecule comprises anti-LIR scFv; C: a NK inhibitory molecule comprises anti-NKG2A scFv, a HLA-G extracellular region, a HLA-E extracellular region or an E-Cad extracellular region.
Figure 6 shows the killing effect of CD4+ T cells (A) and CD8+ T cells (B) expressing NK inhibitory molecules comprising CD3 ζ on NK cells.
Figure 7 shows the expression levels of scFv on CAR7-NKi-dKO T cells and CAR7-NKi-tKO T cells.
Figure 8 shows the expression levels of NK inhibitory molecules of CAR7-NKi-dKO T cells and CAR7-NKi-tKO T cells.
Figure 9 shows the killing ability of CAR7-NKi-dKO T cells and CAR7-NKi-tKO T cells on target cells.
Figure 10 shows the cytokine release levels after CAR7-NKi-dKO T cells and CAR7-NKi-tKO T cells were co-cultured with target cells.
Figure 11 shows the expression levels of scFv on CAR7-E T cells, CAR7-PDL1 T cells and CAR7-EPDL1 T cells.
Figure 12 shows the expression level of HLA-E in CAR7-E T cells and the expression level of PDL1 in CAR7-PDL1 T cells.
Figure 13 shows the expression levels of HLA-E and PDL1 in CAR7-EPDL1 T cells.
Figure 14 shows the killing ability of CAR7-E T cells, CAR7-PDL1 T cells and CAR7-EPDL1 T cells on target cells.
Figure 15 shows the cytokine release levels after CAR7-E T cells, CAR7-PDL1 T cells and CAR7-EPDL1 T cells were co-cultured with target cells.
Figure 16 shows the expression levels of CD7 scFv and CD19 scFv in CAR7-19 T cells.
Figure 17 shows the killing ability of CAR7-19 T cells on two target cells.
Figure 18 shows the cytokine release levels after CAR7-19 T cells were co-cultured with two kinds of target cells.

### Example

### Example 1. Preparation of universal CAR T cells against CD7

Sequences encoding the following proteins were synthesized and cloned sequentially into the pLVX vector (Public Protein/Plasmid Library (PPL), Cat. No.: PPL00157-4a): CD8α signal peptide (SEQ ID NO: 36), anti-CD7 scFv (SEQ ID NO: 21), CD8α hinge region (SEQ ID NO: 38), CD8α transmembrane region (SEQ ID NO: 24), 4-1BB intracellular region (SEQ ID NO: 28), CD3 ζ intracellular signaling domain (SEQ ID NO: 30), and the correct insertion of the target sequences was confirmed by sequencing.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000g, 4°C, 2.5 hours) to obtain concentrated lentiviruses.

Wild-type T cells were activated with DynaBeads CD3/CD28 CTS^{™} (Gibco, Cat. No. 40203D) and cultured at 37°C and 5% CO₂ for 1 day. Then the TCR/CD3 component (specifically TRAC gene), CD7 gene and optional MHC-II related gene (specifically RFX5) in the wild-type T cells were knocked out using the CRISPR system to obtain TCR/CD7 double knockout dKO-T cells and TCR/CD7/RFX5 triple knockout tKO-T cells. Wild-type T cells without gene knockout (i.e., NT cells) were used as controls.

Using FITC Mouse Anti-Human CD3 (BD Pharmingen, Cat. No. 555916) antibody, PE mouse anti-human CD7 (biolegend Cat. No. 395604) and APC anti-human DR, DP, DQ (biolegend, Cat. No. 361714) antibodies, the gene editing efficiency of TCR/CD7/RFX5 in T cells was detected by flow cytometry, and the results are shown in Table 1.

**Table 1. Gene expression efficiency in T cells**

| Name | TCR/CD3 | CD7 | RFX5/MHC-II |
|---|---|---|---|
| dKO-T | 2.2% | 5.3% | 92% |
| tKO-T | 3% | 8.9% | 11.2% |
| NT | 98% | 94% | 92% |

It can be seen from Table 1 that the expression of related genes in dKO-T cells and tKO-T cells prepared in the present disclosure is effectively suppressed or silenced.

The concentrated lentivirus was added to the dKO-T cells and tKO-T cells to obtain CAR7-dKO T cells and CAR7-tKO T cells. Using Biotin-SP (long spacer) AffiniPure Goat Anti-human IgG, F(ab')₂ Fragment Specific (min X Hu, Bov, Hrs Sr Prot) (jackson immunoresearch, Cat. No. 109-065-097) as the primary antibody, APC Streptavidin (BD Pharmingen, Cat. No. 554067) or PE Streptavidin (BD Pharmingen, Cat. No. 554061) as the secondary antibody, the expression levels of scFv on the CAR7-dKO T cells and CAR7-tKO T cells were detected by flow cytometry, and the results are shown in Figure 2.

It can be seen that the scFv in the CAR T cells prepared by the present disclosure can be effectively expressed.

### Example 2. Killing effect of CAR T cells on target cells and release of cytokines thereof

### 2.1 Killing effect of CAR-T cells on target cells

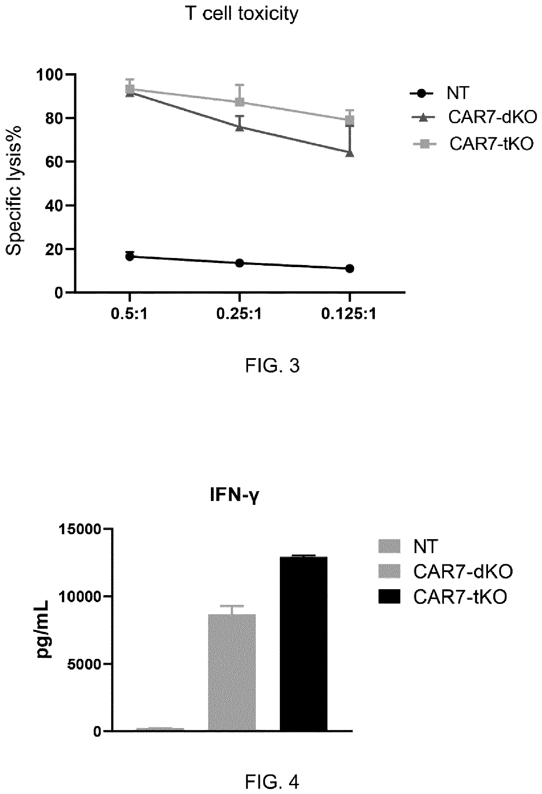

In order to detect the killing ability of CAR-T cells on target cells, firstly Jurkat target cells carrying the fluorescein gene were plated into a 96-well plate at 1×10⁴/well, and then CAR T cells and NT cells were plated into the 96-well plate with effector-target ratios (i.e., the ratio of effector T cells to target cells) of 0.5:1, 0.25:1 and 0.125:1 for co-culture, and the fluorescence value was measured with a microplate reader after 16-18 hours. According to the calculation formula: (average fluorescence value of target cells - average fluorescence value of samples)/average fluorescence value of target cells × 100%, the killing efficiency was calculated, and the results are shown in Figure 3.

It can be seen that compared with NT, both CAR7-dKO T cells and CAR7-tKO T cells have specific killing ability on target cells, and the killing ability of CAR7-tKO T cells is higher than that of CAR7-dKO T cells.

### 2.2 Cytokine release of CAR-T cells

When T cells kill target cells, cytokines are released while the number of target cells is reduced. According to the following steps, enzyme-linked immunosorbent assay (ELISA) was used to measure the release level of cytokine IFNγ when the CAR T cells of the present disclosure kill target cells.

### (1) Collection of cell co-culture supernatant

Jurkat target cells were plated in a 96-well plate at 1×10⁵/well, and then CAR T and NT cells (negative control) were co-cultured with the target cells at a ratio of 0.125:1, and the cell co-culture supernatant was collected after 18-24 hours.

### (2) Detection of the secretion of IFN γ in the supernatant by ELISA

A 96-well plate was coated with Purified anti-human IFN-γ Antibody (Biolegend, Cat. No. 506502) as capture antibody and incubated overnight at 4°C, and then the antibody solution was removed. 250 µL of PBST (1XPBS comprising 0.1% Tween) solution comprising 2% BSA (sigma, Cat. No. V900933-1kg) was added, and incubated at 37°C for 2 hours. The plate was then washed 3 times with 250 µL of PBST (1XPBS comprising 0.1% Tween). 50 µL of cell co-culture supernatant or standard per well was added and incubated at 37 °C for 1 hour, then the plate was washed 3 times with 250 µL of PBST (1XPBS comprising 0.1% Tween). Then 50 µL of Anti-Interferon gamma antibody [MD-1] (Biotin) (abcam, Cat. No. ab25017) as detection antibody, was added to each well, incubated at 37°C for 1 hour, and the plate was washed 3 times with 250 µL of PBST (1XPBS comprising 0.1% Tween). Then HRP Streptavidin (Biolegend, Cat. No. 405210) was added, incubated at 37°C for 30 minutes, and the supernatant was discarded. 250 µL of PBST (1XPBS comprising 0.1% Tween) was added for washing 5 times. 50 µL of TMB substrate solution was added to each well. Reactions were allowed to occur at room temperature in the dark for 30 minutes, after which 50 µL of 1 mol/L H₂SO₄ was added to each well to terminate the reaction. Within 30 minutes after the reaction termination, a microplate reader was used to detect the absorbance at 450 nm, and the content of cytokines was calculated according to the standard curve (drawn according to the reading value and concentration of the standard), the results are shown in Figure 4.

It can be seen that the cytokine release of CAR7-dKO T cells and CAR7-tKO T cells to target cells was significantly higher than that of the control NT group, and the release level of CAR7-tKO T cells was higher than that of CAR7-dKO T cells.

From the above results, it can be seen that the killing ability of CAR7-tKO T cells on target cells and the release level of cytokines are higher than those of CAR7-dKO T cells, indicating that knockout of MHC-II genes enhances CAR-T cell killing activity. This is unexpected, because existing reports generally believe that the expression of MHC-II genes is related to immune rejection, and there has not been any report on the relationship between MHC-II genes and the activity of CAR-T cells themselves.

### Example 3. Inhibitory effect of NK inhibitory molecules on NK cell killing activity

The coding sequences of the following proteins were synthesized and cloned sequentially into the pLVX vector (Public Protein/Plasmid Library (PPL), Cat. No.: PPL00157-4a): B2m signal peptide (SEQ ID NO: 34), NK inhibitory ligand, CD28 hinge region (SEQ ID NO: 40), CD28 transmembrane region (SEQ ID NO: 24), wherein the NK inhibitory ligand is an extracellular region of E-cadherin (SEQ ID NO: 47, corresponding to the ECad0 plasmid), a fusion molecule of B2M and HLA-E extracellular region (comprising presenting peptide SEQ ID NO: 75, B2M SEQ ID NO: 74 and HLA-E extracellular region mutant SEQ ID NO: 51, wherein the coding sequence of B2M, SEQ ID NO: 82, comprises a synonymous mutation, corresponding to the E0 plasmid) or a fusion molecule of B2M and HLA-G extracellular region (comprising B2M SEQ ID NO: 74 and HLA-G extracellular region SEQ ID NO: 49, wherein the coding sequence of B2M, SEQ ID NO: 82, comprises a synonymous mutation, corresponding to the G0 plasmid). The CD28 co-stimulatory domain (SEQ ID NO: 26) was further included in the ECad0, E0 and G0 plasmids to obtain the ECad28, E28 and G28 plasmids, respectively. The correct insertion of the target sequences in the plasmid was confirmed by sequencing.

The coding sequences of the following proteins were synthesized and cloned sequentially into the pLVX vector (Public Protein/Plasmid Library (PPL), Cat. No.: PPL00157-4a): B2m signal peptide (SEQ ID NO: 34), NK inhibitory ligand, IgG4 hinge region (SEQ ID NO: 42), CD8α transmembrane region (SEQ ID NO: 22), CD28 co-stimulatory domain (SEQ ID NO: 26), wherein the NK inhibitory ligand is anti-NKG2A scFv (SEQ ID NO: 63, corresponding to A28 plasmid), anti-KIR scFv (SEQ ID NO: 67, corresponding to KIRG4 plasmid) or anti-LIR1 scFv (SEQ ID NO: 70, corresponding to LIR1-1 plasmid). The correct insertion of the target sequences in the plasmid was confirmed by sequencing.

The coding sequences of the following proteins were synthesized and cloned sequentially into the pLVX vector (Public Protein/Plasmid Library (PPL), Cat. No.: PPL00157-4a): CD8α signal peptide (SEQ ID NO: 36), anti-LIR1 scFv (SEQ ID NO: 73), CD28 hinge region (SEQ ID NO: 40), CD28 transmembrane region (SEQ ID NO: 24), 4-1BB co-stimulatory domain (SEQ ID NO: 28), to obtain a LIR1-2 plasmid. The correct insertion of the target sequences in the plasmid was confirmed by sequencing.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000g, 4°C, 2.5 hours) to obtain concentrated lentiviruses.

T cells were activated with DynaBeads CD3/CD28 CTSTM (Gibco, Cat. No. 40203D) and cultured at 37°C and 5% CO₂ for 1 day. Then, the concentrated lentivirus was added, and after continuous culture for 3 days, T cells expressing NK inhibitory molecules, i.e., UNKi-T cells, were obtained.

Then the TCR/CD3 component (specifically TRAC gene) and MHC-related genes (specifically B2M and RFX5) in wild-type T cells (Mock T cells, used as a control) and the UNKi-T cells were knocked out using the CRISPR system, and it is confirmed that each gene was effectively knocked out by flow cytometry.

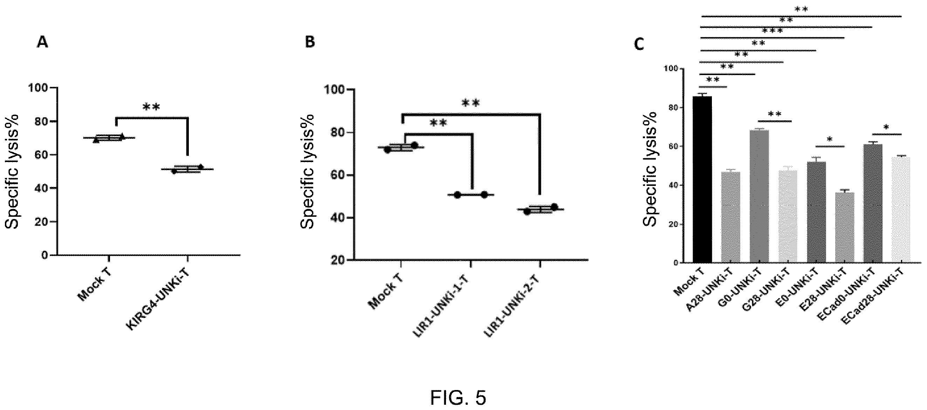

Then, the inhibitory effect of the UNKi-T cells prepared in the present disclosure on the killing effect of NK cells was detected according to the following method: the UNKi-T cells and Mock-T cells prepared in the present disclosure were labeled with Far-Red (invitrogen, Cat. No. C34564). Then the labeled UNKi-T cells and Mock T cells were plated into 96-well plates at a concentration of 1×10⁴ cells/well, and NK92 cells (for UNKi-T cells expressing HLA-E extracellular region, HLA-G extracellular region, anti-NKG2A scFv, anti-KIR scFv or anti-LIR1 scFv and Mock T cells) or NK92-KLRG1 cells (for UNKi-T cells expressing E-cadherin extracellular region, prepared by introducing the KLRG1 gene into NK92 cells) were added at an effector-target ratio of 2:1 for co-culture. After 16-18 hours, the proportion of T cells in the culture was detected by flow cytometry, and then the killing effect of NK cells on T cells was calculated. The results are shown in Figure 5.

It can be seen from Figure 5 that compared with Mock T cells that do not express NK inhibitory molecules, UNKi-T cells expressing inhibitory ligands such as anti-KIR scFv, anti-LIR1 scFv, anti-NKG2A scFv, HLA-G extracellular region, HLA-E extracellular region and E-cadherin extracellular region can significantly reduce the killing effect of NK cells on T cells. In addition, compared with T cells (G0, E0, Ecad0) expressing only an inhibitory ligand and a transmembrane domain (i.e., not comprising a co-stimulatory domain), the addition of the co-stimulatory domain can further significantly enhance the inhibition of killing effect of NK cell on T cells (G28, E28, ECad28). Therefore, the NK inhibitory molecules comprising an inhibitory ligand, a transmembrane domain and a co-stimulatory domain of the present disclosure can significantly reduce the killing effect of NK cells on UNKi-T cells, thereby effectively reducing the risk of HvGD.

In addition, in some cases, it is not only necessary to inhibit the killing effect of NK cells on CAR-T cells, but even further requires T cells to kill NK cells. Therefore, the inventors further included the CD3 ζ intracellular signaling domain (SEQ ID NO: 30) on the basis of the E28 plasmid and A28 plasmid cells, and packaged them as lentivirus according to the above method, and infected T cells in which TCR/CD3 components (specifically TRAC gene) and MHC-related genes (specifically B2M and RFX5) were effectively knocked out, to obtain E28z-UNKi-T cells and A28z-UNKi-T cells.

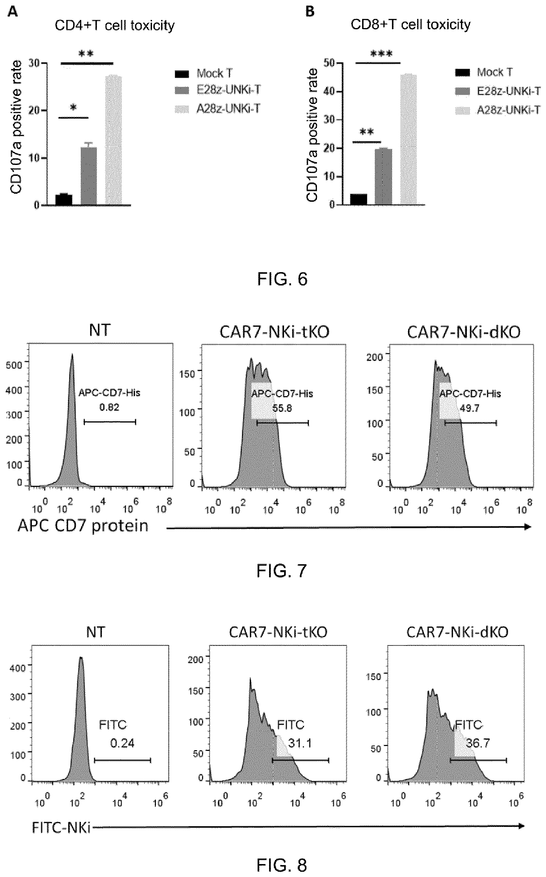

The killing of NK cells by UNKi-T cells was detected by the following method: the target cells (NK92 cells) were plated in a 96-well plate at a concentration of 1×10⁵ cells/well, and then Mock T cells, E28z-UNKi-T cells and A28z-UNKi-T cells were added to each well at a ratio of 1:1, and at the same time, 10 µl of PE-anti-human CD107a (BD Pharmingen, Cat. No. 555801) was added for co-culturing at 37°C and 5% CO₂. After 1 hour, Goigstop (BD Pharmingen, Cat. No. 51-2092KZ) was added to continue incubation for 2.5 hours. Then 5 µl APC-anti human CD8 (BD Pharmingen, Cat. No.: 555369) and 5 µl FITC-anti human CD4 (BD Pharmingen, Cat. No.: 561005) were added to each well, and incubated at 37°C for 30 minutes. The expression of CD107a was detected by flow cytometry, and the results are shown in Figure 6A (CD4+ T cell toxicity) and Figure 6B (CD8+ T cell toxicity).

It can be seen that Mock T cells that do not express NK inhibitory molecules hardly kill target cells. On the contrary, after the E28z-UNKi-T cells and A28z-UNKi-T cells prepared by the present disclosure were co-cultured with the target cells, the expression rate of CD107a was significantly increased, indicating that the UNKi-T cells of the present disclosure can significantly kill NK cells.

### Example 4. Preparation of universal CAR T cells expressing NK inhibitory molecules

Sequences encoding the following proteins were synthesized and cloned into the MSCV vector: CD8α signal peptide (SEQ ID NO: 36), anti-CD7 scFv (SEQ ID NO: 21), CD8α hinge region (SEQ ID NO: 38), CD8α transmembrane region (SEQ ID NO: 22), 4-1BB intracellular region (SEQ ID NO: 28), CD3 ζ intracellular signaling domain (SEQ ID NO: 30), F2A, E-cadherin extracellular region (SEQ ID NO: 48), CD28 hinge region (SEQ ID NO: 40), CD28 transmembrane region (SEQ ID NO: 24) and CD28 intracellular region (SEQ ID NO: 26), and the correct insertion of the target sequences was confirmed by sequencing.

Three ml Opti-MEM (Gibco, Cat. No. 31985-070) was added to a sterile tube to dilute the above plasmid, and then packaging vector pCL-Eco (Shanghai Hewu Biotechnology Co., Ltd., Cat. No. P3029) was added according to the ratio of plasmid : viral packaging vector = 3:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask of 293 GP cells. Viruses were collected at 72 hours and 96 hours, pooled, centrifuged (2000rpm, 4°C, 10 min) to remove fragments and obtain retroviral supernatant.

TCR/CD7 double-knockout dKO T cells and TCR/CD7/RFX5 triple-knockout tKO T cells were prepared according to the knockout method in Example 1. Wild-type T cells without gene knockout (i.e., NT cells) were used as controls.

A 24-well plate was coated with Retronectin and incubated overnight at 4°C. Then the solution was removed, and 300 µL of PBS solution comprising 5% FBS (Gibco, Cat. No.) was added and left at room temperature for 30 min. The supernatant was then removed and the plate was washed 2 times with 1 mL of PBS. 2mL retrovirus supernatant and 0.5M dKO-T cells or tKO-T cells were added to each well, centrifuged at 2000g and 32°C for 2h and cultured in a carbon dioxide incubator to obtain CAR7-NKi-dK0 T cells and CAR7-NKi - tKO T cells.

After 7 days of culture, using Biotin-SP (long spacer) AffiniPure Goat Anti-Mouse IgG, F(ab')₂ Fragment Specific (min X Hu, Bov, Hrs Sr Prot) (jackson immunoresearch, Cat. No. 115-065-072) as the primary antibody, APC Streptavidin (BD Pharmingen, Cat. No. 554067) or PE Streptavidin (BD Pharmingen, Cat. No. 554061) as the secondary antibody, the expression levels of CD7 scFv in the CAR7-NKi-dK0 T cells and CAR7-NKi-tK0 T cells were detected by flow cytometry, and the results are shown in Figure 7. The expression of E-Cadherin in CART cells was detected using E-cadherin monoclonal antibody (Invitrogen, Cat. No. 13-5700) and Goat anti-Mouse IgG (H+L) Cross-Adsorbed Secondary Antibody, Alexa Fluor 488 (Invitrogen, Cat. No. A-11001), and the results are shown in Figure 8.

It can be seen that both anti-CD7 scFv and NKi inhibitory molecules can be effectively expressed in the CAR T cells prepared in the present disclosure.

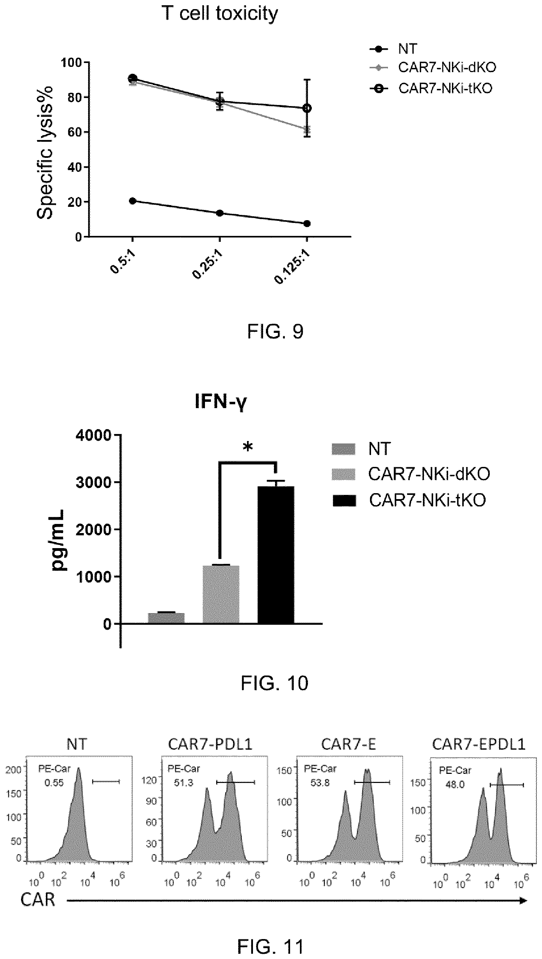

The killing effect of CAR7-NKi-dK0 T cells and CAR7-NKi-tK0 T cells on Jurkat target cells was detected according to the method described in 2.1 of Example 2, and the results are shown in Figure 9. It can be seen that the two CAR-T cells can significantly kill target cells at various effector-target ratios, and at the effector-target ratio of 0.125:1, the killing effect of CAR7-NKi-tK0 T cells is better than that of CAR7-NKi -dKO T cells.

The cytokine release levels after CAR7-NKi-dK0 T cells and CAR7-NKi-tK0 T cells were co-cultured with Jurkat target cells were detected according to the method described in 2.2 of Example 2, and the results are shown in Figure 10. It can be seen that the cytokine release levels of the CAR7-NKi-dK0 T cells and CAR7-NKi-tK0 T cells of the present disclosure were significantly higher than those of the control NT cells, and the release level of CAR7-NKi-tKO T cell group was significantly higher than that of CAR7-NKi-dK0 T cell group.

### Example 5. Preparation of universal CAR T cells expressing NK inhibitory molecules and verification of their functions

Sequences encoding the following proteins were synthesized and cloned into the MSCV vector: CD8α signal peptide (SEQ ID NO: 36), anti-CD7 scFv (SEQ ID NO: 21), CD28 hinge region (SEQ ID NO: 40), CD8α transmembrane region (SEQ ID NO: 22), CD28 intracellular region (SEQ ID NO: 26), CD3 ζ intracellular signaling domain (SEQ ID NO: 32), F2A, PD-L1 signal peptide (SEQ ID NO: 44), PD-L1 extracellular region (SEQ ID NO: 45), CD28 transmembrane region (SEQ ID NO: 24), 4-1BB intracellular region (SEQ ID NO: 28), and the correct insertion of the target sequences was confirmed by sequencing (plasmid designation: CAR7-PDL1).

Sequences encoding the following proteins were synthesized and cloned into the MSCV vector: CD8α signal peptide (SEQ ID NO: 36), anti-CD7 scFv (SEQ ID NO: 21), CD8α hinge region (SEQ ID NO: 38), CD28 transmembrane region (SEQ ID NO: 24), 4-1BB intracellular region (SEQ ID NO: 28), CD3 ζ intracellular signaling domain (SEQ ID NO: 32), F2A, B2M signal peptide (SEQ ID NO: 34), HLA-E extracellular region (SEQ ID NO: 50), CD28 transmembrane region (SEQ ID NO: 24), CD28 intracellular region (SEQ ID NO: 26), and the correct insertion of the target sequences was confirmed by sequencing ( Plasmid designation: CAR7-E).

Sequences encoding the following proteins were synthesized and cloned into the MSCV vector: CD8α signal peptide (SEQ ID NO: 36), anti-CD7 scFv (SEQ ID NO: 21), CD8α hinge region (SEQ ID NO: 38), CD8α transmembrane region (SEQ ID NO: 22), 4-1BB intracellular region (SEQ ID NO: 28), CD3 ζ intracellular signaling domain (SEQ ID NO: 32), F2A, B2M signal peptide (SEQ ID NO: 34 ), HLA-E extracellular region (SEQ ID NO: 50), linker peptide (SEQ ID NO: 64), PD-L1 extracellular region (SEQ ID NO: 45), CD28 transmembrane region (SEQ ID NO: 24 ), CD28 intracellular region (SEQ ID NO: 26), and the correct insertion of the target sequences was confirmed by sequencing (plasmid designation: CAR7-EPDL1).

According to the method described in Example 3, the above plasmids were packaged into retroviruses and infected into tKO-T cells to obtain CAR7-E T cells, CAR7-PDL1 T cells and CAR7-EPDL1 T cells, respectively.

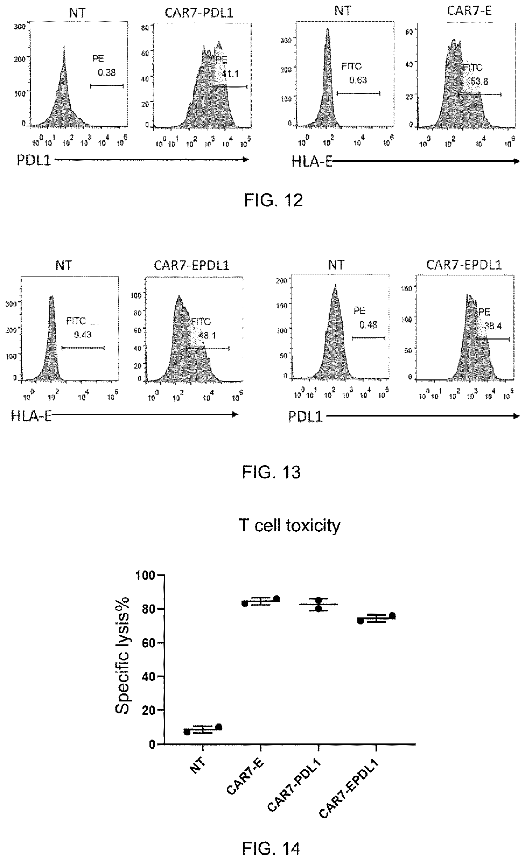

After 7 days of culture, using Biotin-SP (long spacer) AffiniPure Goat Anti-human IgG, F(ab')₂ Fragment Specific (min X Hu, Bov, Hrs Sr Prot) (jackson immunoresearch, Cat. No. 109-065-097) as the primary antibody, APC Streptavidin (BD Pharmingen, Cat. No. 554067) or PE Streptavidin (BD Pharmingen, Cat. No. 554061) as the secondary antibody, the expression levels of scFv in the three cells were detected by flow cytometry, and the results are shown in Figure 11. The expression of HLA-E and PDL1 in CAR T cells was detected using PE mouse anti-human HLA-E (biolegend, Cat. No. 342604) and PE anti-human PDL1 (biolegend, Cat. No. 329706), respectively, and the results are shown in Figure 12 and Figure 13.

It can be seen that both anti-CD7 scFv and NK inhibitory molecules (HLA-E and PD-L1) can be effectively expressed in the CAR T cells prepared in the present disclosure.

According to the method described in 2.1 of Example 2, the killing effect of three CAR-T cells on Jurkat target cells was detected, and the results are shown in Figure 14. It can be seen that all three CAR-T cells can significantly kill target cells.

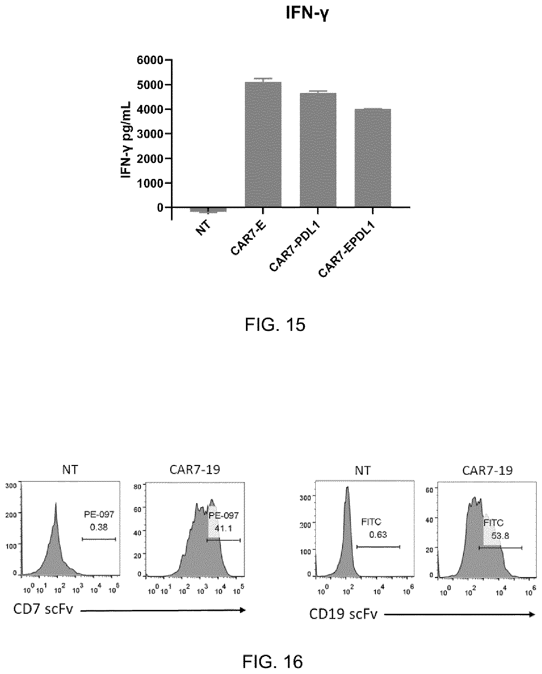

According to the method described in 2.2 of Example 2, the cytokine release levels after the three CAR-T cells were co-cultured with Jurkat target cells were detected, and the results are shown in Figure 15. Compared with the NT group, the cytokine release levels of the three CAR-T cells were significantly increased.

### Example 6. Preparation of dual-target CAR-T cells and verification of their functions

Sequences encoding the following proteins were synthesized and cloned into the pLVX vector (Public Protein/Plasmid Library (PPL), Cat. No.: PPL00157-4a): CD8α signal peptide (SEQ ID NO: 36), anti-CD7 scFv ( SEQ ID NO: 20), linker peptide (SEQ ID NO: 64), anti-CD19 scFv (SEQ ID NO: 54), CD8α hinge region (SEQ ID NO: 38), CD8α transmembrane region (SEQ ID NO: 22), 4-1BB intracellular region (SEQ ID NO: 28), CD3 ζ intracellular signaling domain (SEQ ID NO: 32), and the correct insertion of the target sequences was confirmed by sequencing.

According to the method of Example 1, the above plasmid vectors were packaged into lentivirus, and infected into tkO-T cells to obtain CAR7-19 T cells. Unmodified wild-type T cells were used as negative controls (NT).

Using Biotin-SP (long spacer) AffiniPure Goat Anti-Mouse IgG, F(ab')₂ Fragment Specific (min X Hu, Bov, Hrs Sr Prot) (jackson immunoresearch, Cat. No. 115-065-072) or Biotin -SP (long spacer) AffiniPure Goat Anti-human IgG, F(ab')₂ Fragment Specific(min X Hu, Bov, Hrs Sr Prot) (jackson immunoresearch, Cat. No. 109-065-097) as the primary antibody, FITC Streptavidin ( BD Pharmingen, Cat. No. 554060) or PE Streptavidin (BD Pharmingen, Cat. No. 554061) as the secondary antibody, the expression level of scFv in CAR7-19 T cells was detected by flow cytometry, and the results are shown in Figure 16.

It can be seen that both CD7 scFv and CD19 scFv can be effectively expressed in the CAR T cells prepared in the present disclosure.

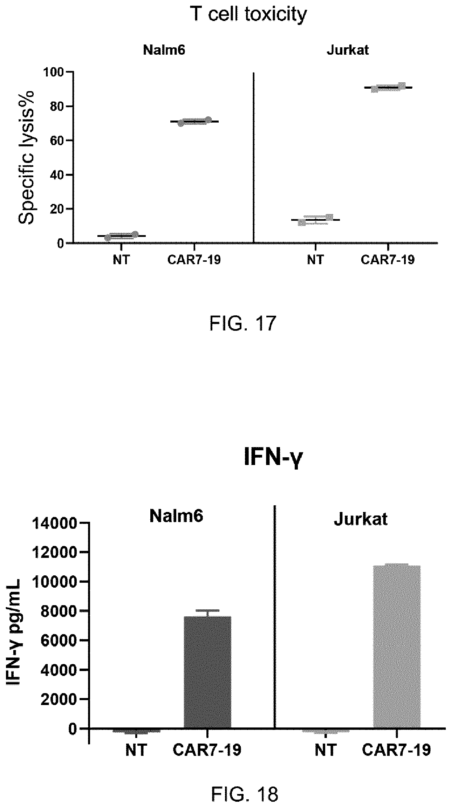

According to the method in Example 2, the killing function and cytokine release level of CAR7-19 T cells were detected, and the results are shown in Figure 17 and Figure 18, respectively. It can be seen that CAR7-19 T cells have specific killing effect on both Nalm6 and Jurkat target cells and significantly increased cytokine release.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. An engineered immune cell, (1) expressing a chimeric antigen receptor comprising an antigen-binding region, the antigen-binding region comprising an anti-CD7 antibody; and (2) having suppressed or silenced expression of endogenous CD7, at least one TCR/CD3 gene, and at least one MHC-II related gene.

2. The engineered immune cell according to claim 1, **characterized in that** the chimeric antigen receptor comprises the anti-CD7 antibody, a transmembrane domain, and an intracellular signaling domain.

3. The engineered immune cell according to claim 1 or 2, **characterized in that** the anti-CD7 antibody comprises CDR-L1, CDR-L2 and CDR-L3 as set forth in SEQ ID NOs: 1, 2 and 3 respectively, and CDR-H1, CDR-H2 and CDR-H3 as set forth in SEQ ID NOs: 4, 5 and 6 respectively.

4. The engineered immune cell according to any one of claims 1-3, **characterized in that** the antigen-binding region of the chimeric antigen receptor further comprises an antibody targeting a second antigen, or a functional fragment thereof, wherein the second antigen is selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof.

5. The engineered immune cell according to any one of claims 1-4, **characterized in that** the chimeric antigen receptor comprises an anti-CD7 antibody and an anti-CD19 antibody.

6. The engineered immune cell according to any one of claims 1-5, **characterized in that** the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154.

7. The engineered immune cell according to any one of claims 1-6, **characterized in that** the intracellular signaling domain is an intracellular region of a protein selected from the group consisting of: FcR γ, FcR β, CD3 γ, CD3 δ, CD3 ε, CD3 ζ, CD22, CD79a, CD79b, and CD66d.

8. The engineered immune cell according to any one of claims 1-7, **characterized in that** the chimeric antigen receptor further comprises at least one co-stimulatory domain, which is an intracellular region of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70 and a combination thereof.

9. The engineered immune cell according to claim 1, **characterized in that** the TCR/CD3 gene is selected from the group consisting of TRAC, TRBC, CD3 γ, CD3 δ, CD3 ε, CD3 ζ and a combination thereof.

10. The engineered immune cell according to claim 1, **characterized in that** the MHC-II related gene is selected from the group consisting of: HLA-DPA, HLA-DQ, HLA-DRA, RFX5, RFXAP, RFXANK, CIITA and a combination thereof.

11. The engineered immune cell according to any one of claims 1-10, **characterized in that** the engineered immune cell has suppressed or silenced expression of endogenous CD7, at least one TCR/CD3 gene selected from the group consisting of TRAC and TRBC and at least one MHC class II gene selected from the group consisting of RFX5, RFXAP, RFXANK and CIITA.

12. The engineered immune cell according to any one of claims 1-11, **characterized in that** the engineered immune cell further expresses a NK inhibitory molecule, and the NK inhibitory molecule comprises one or more NK inhibitory ligands, a transmembrane domain and at least one co-stimulatory domain.

13. The engineered immune cell according to claim 12, **characterized in that** the NK inhibitory ligand is an antibody targeting a NK inhibitory receptor or a functional fragment thereof, wherein the NK inhibitory receptor is preferably selected from the group consisting of NKG2A, NKG2B, CD94, LIR1, LIR2, LIR3, LIR5, LIR8, KIR2DL1, KIR2DL2/3, KIR2DL5A, KIR2DL5B, KIR3DL1, KIR3DL2, KIR3DL3, CEACAM1, LAIR1, NKR-P1B, NKR-P1D, PD-1, TIGIT, CD96, TIM3, LAG3, SIGLEC7, SIGLEC9, Ly49A, Ly49C, Ly49F, Ly49G1, Ly49G4 and KLRG1.

14. The engineered immune cell according to claim 12, **characterized in that** the NK inhibitory ligand is HLA-E, HLA-F, HLA-G, cadherin, collagen, OCIL, sialic acid, PD-L1/PD-L2, CTLA-4, CD155, CD112, CD113, Gal-9, FGL1, or a NK inhibitory receptor binding region thereof.

15. The engineered immune cell according to claim 12, **characterized in that** the NK inhibitory molecule further comprises a CD3 ζ intracellular region as the intracellular signaling domain.

16. The engineered immune cell according to any one of claims 1-15, **characterized in that** the engineered immune cell is a T cell, a macrophage, a dendritic cell, a monocyte, a NK cell or a NKT cell.

17. A pharmaceutical composition comprising the engineered immune cell according to any one of claims 1-16, and one or more pharmaceutically acceptable excipients.

18. Use of the engineered immune cell according to any one of claims 1-16 or the pharmaceutical composition according to claim 17 in the preparation of a medicine for treating a disease related to CD7 expression.
